# EUROPÄISCHE PATENTANMELDUNG

(11) **EP 0 928 814 A2**
(43) Veröffentlichungstag der Anmeldung: **14.07.1999**
(21) Anmeldenummer: 98124150.8
(22) Anmeldetag: 19.12.1998
(51) Int. Cl.: C09B 19/02, C07D 498/22

(54) **Verfahren zur Herstellung von Dioxazin-Verbindungen**

(30) Priorität: 07.01.1998 DE 19800273
(71) Anmelder: Clariant GmbH, 65929 Frankfurt am Main (DE)
(72) Erfinder: Kurz, Hans, Dr., 61184 Karben (DE); Stoll, Matthias, 63628 BSS-Hausen (DE); Nagl, Gert, Dr., 61338 Niederdorfelden (DE)

(57) **Zusammenfassung**

Die vorliegende Erfindung betrifft ein Verfahren zur Herstellung von Dioxazin-Derivaten durch Ringschluß einer Verbindung der allgemeinen Formel (II) 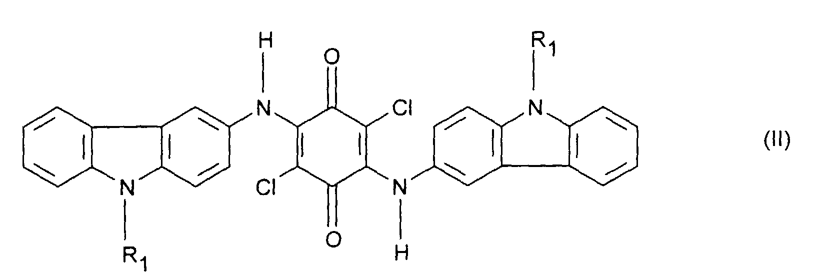 worin R¹ Wasserstoff oder (C₁-C₈)-Alkyl bedeutet, in Gegenwart eines Ringschlußmittels, dadurch gekennzeichnet, daß als Ringschlußmittel Chlor eingesetzt wird.
Dioxazin-Verbindungen werden zur Herstellung wertvoller Farbstoffe und Pigmente verwendet.

## Beschreibung

Die vorliegende Erfindung betrifft ein verbessertes Verfahren zur Herstellung von Dioxazin-Verbindungen.

Dioxazin-Verbindungen werden zur Herstellung wertvoller Farbstoffe und Pigmente verwendet (s. Ullmann's Encyclopedia of Industrial Chemistry, Vol. A 3, p. 233) und können technisch beispielsweise durch Ringschluß von Verbindungen der allgemeinen Formel II 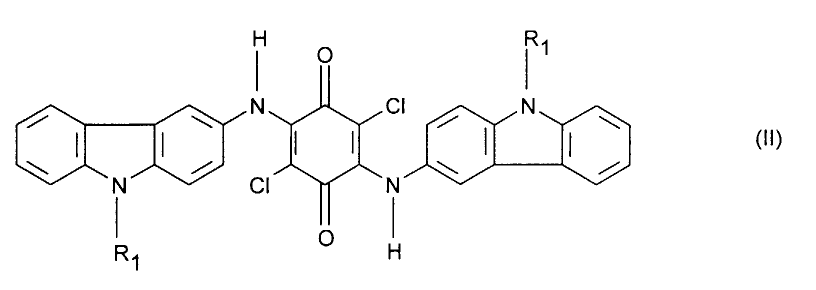 mit Benzolsulfonsäurechlorid oder 4-Toluolsulfonsäurechlorid als Ringschlußmittel hergestellt werden (siehe DE-A 30 10 949).

Als Ringschlußmittel wurden außerdem Benzolsulfonsäurechloride vorgeschlagen, die am Benzolring elektronenziehende Substituenten tragen (siehe JP Hei 7-145327). Auch der Ringschluß unter vermindertem Druck wurde bereits beschrieben (siehe JP Hei 5-43808).

Nachteilig ist bei diesen Methoden, bei denen als Ringschlußmittel Benzolsulfonsäurechlorid oder dessen Derivate eingesetzt werden, die Tatsache, daß aus dem Ringschlußmittel neben Chlorwasserstoff eine große Menge an nicht verwertbarem organischen Abfall entsteht. Der daraus resultierende Verbesserungsbedarf im Hinblick auf Schonung der Resourcen wurde bereits erkannt.

So werden in der JP Sho 62-192385 zu diesem Zweck als Ringschlußmittel heterocyclische Amine vorgeschlagen, die zurückgewonnen werden können. Dadurch entfällt zwar der aus dem Ringschlußmittel entstehende Abfall. Die so hergestellten Produkte enthielten jedoch wesentlich weniger Chlor als sich nach der Summenformel errechnet. Sie entsprechen somit nicht dem gewünschten Produkt. Es stellt sich deshalb die Aufgabe, ein Ringschluß-Verfahren für die Herstellung von Dioxazin-Verbindungen bereitzustellen, das folgende Kriterien erfüllt:
Statt eines organischen Ringschlußmittels soll zur Schonung der Resourcen ein billiges anorganisches Ringschlußmittel verwendet werden.
Bei dem Ringschluß soll der aus organischen Ringschlußmitteln entstehende Abfall vermieden werden.
Das Produkt soll in einer gut filtrierbaren Form anfallen, sehr rein sein und im Finish ein Pigment mit hoher Farbstärke, reinem Farbton und sehr guten Echtheitseigenschaffen ergeben.

Es wurde überraschenderweise gefunden, daß sich Dioxazin-Verbindungen unter Erfüllung der genannten Kriterien herstellen lassen, wenn als Ringschlußmittel anstelle von Benzolsulfonsäurechlorid oder anstelle von substituierten Benzolsulfonsäurechloriden Chlor verwendet wird.

Die vorliegende Erfindung betrifft somit ein Verfahren zur Herstellung von Dioxazin-Verbindungen durch Ringschluß einer Verbindung der allgemeinen Formel (II) 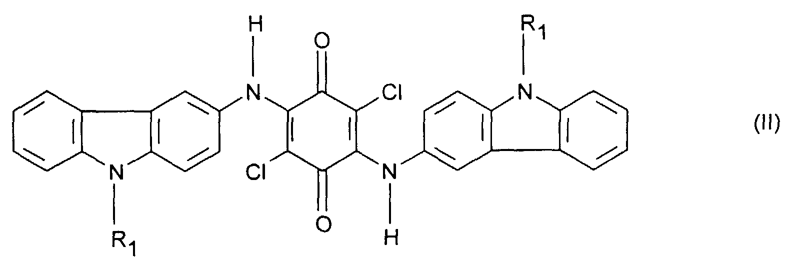 worin R¹ Wasserstoff oder (C₁-C₈)-Alkyl bedeutet, in Gegenwart eines Ringschlußmittels, dadurch gekennzeichnet, daß als Ringschlußmittel Chlor eingesetzt wird.

Für R¹ stehendes (C₁-C₈)-Alkyl kann geradkettig oder verzweigt sein und beispielsweise für Methyl, Ethyl, n-Propyl, i-Propyl, n-Butyl, sek.Butyl, i-Butyl, tert.Butyl, Pentyl, Hexyl, Heptyl oder Octyl stehen. Bevorzugt ist Ethyl.

Die bei den erfindungsgemäßen Verfahren entstehenden Dioxazin-Verbindungen können prinzipiell eine Struktur der Formeln (a), (b) oder (c) besitzen oder eine Mischung dieser Verbindungen sein:

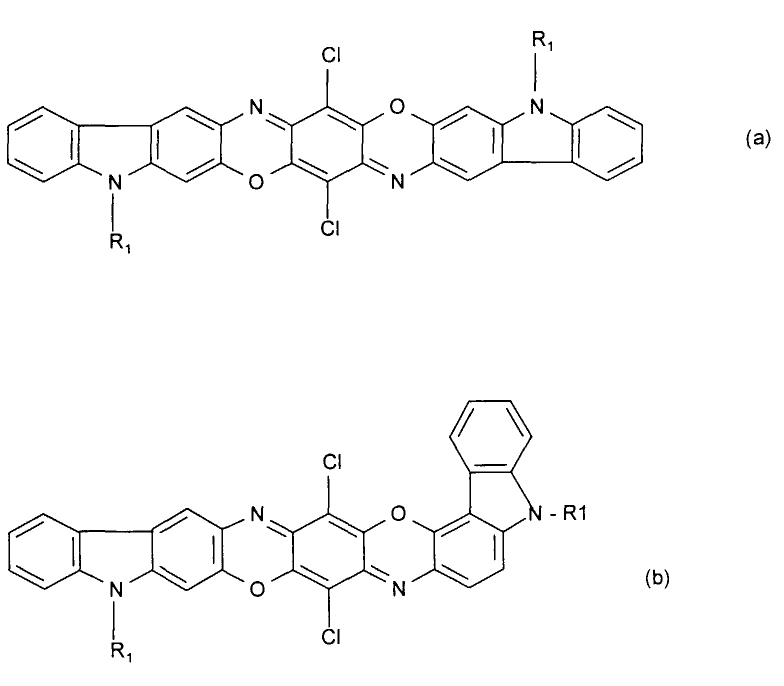

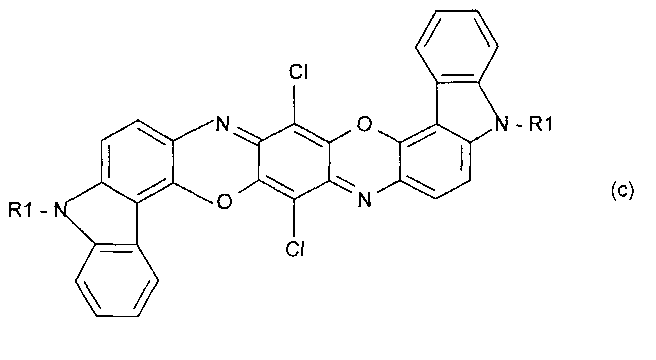

Das Chlor wird bei der Ringschlußreaktion zu Chlorwasserstoff reduziert, der aus dem Reaktionsgemisch entweicht und auf die übliche Weise, z. B. mit Wasser oder verdünnter Natronlauge, absorbiert wird.

Die Chlormenge kann in weiten Grenzen variiert werden. Bevorzugt wird das Chlor in einer Menge von 1,5 bis 5 mol, bezogen auf 1 mol eingesetzte Verbindung der allgemeinen Formel II, zudosiert. Besonders bevorzugt sind Mengen von 2,2 bis 3,5 mol, bezogen auf 1 mol eingesetzte Verbindung der allgemeinen Formel II. Das Chlor wird vorteilhaft während der gesamten Reaktionszeit möglichst tief unter die Oberfläche geleitet. Das Chlor kann mit konstanter Strömungsgeschwindigkeit eingeleitet werden. Der Chlorstrom kann aber auch während der Reaktionszeit nach einer von der Zeit abhängigen Funktion verändert werden. Bevorzugt wird er nach einer Funktion verändert, die mit der Reaktionszeit fallend ist, zum Beispiel nach einer während der Reaktionszeit linear auf den Wert Null abnehmenden Funktion. Vorteilhaft werden in der Praxis auch übliche Annäherungen an solche Funktionen verwendet (s. Ausführungsbeispiele 2 bis 4).

Die erfindungsgemäße Ringschlußreaktion erfolgt bevorzugt bei Temperaturen zwischen 150°C und 200°C. Als Reaktionsmedium sind inerte organische Lösungsmittel mit entsprechend hoher Siedetemperatur geeignet. Geeignet sind beispielsweise Alkylbenzole, Dialkylbenzole, Alkylnaphthaline, Alkane, Alkene, o-Dichlorbenzol, m-Dichlorbenzol oder p-Dichlorbenzol. Bevorzugtes Reaktionsmedium ist o-Dichlorbenzol. Die Reaktion kann auch unter Druck oder unter reduziertem Druck erfolgen. Die Reaktionsgeschwindigkeit ist vergleichbar mit dem konventionellen Ringschluß mit Benzolsulfonsäurechloriden als Ringschlußmittel. Beispielsweise ist in siedendem o-Dichlorbenzol der Ringschluß spätestens nach vier Stunden praktisch beendet.

Am Ende der Reaktionszeit wird das Reaktionsgemisch bevorzugt heiß filtriert und die erhaltene Rohproduktpaste mit heißem Lösungsmittel, bevorzugt mit heißem o-Dichlorbenzol gewaschen. Das anhaftende, mit Wasser nicht mischbare Lösungsmittel wird durch Wasserdampfdestillation, Trocknung oder Auswaschen mit einem sowohl mit dem verwendeten Lösungsmittel als auch mit Wasser mischbaren Lösungsmittel entfernt bzw. verdrängt. Um wasserlösliche Nebenprodukte zu entfernen, wird in der Regel noch mit Wasser gewaschen bzw. extrahiert.

Die Verbindungen der allgemeinen Formel II können beispielsweise in an sich bekannter Weise durch Kondensation von 3-Amino-carbazolen der allgemeinen Formel (III), 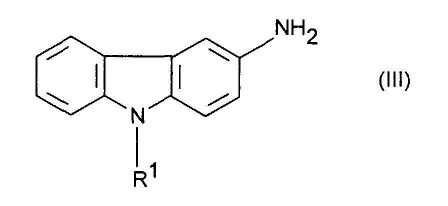 worin R¹ wie oben angegeben definiert ist, mit Chloranil in einem inerten organischen Lösungsmittel hergestellt werden. Als inerte organische Lösungsmittel können dabei beispielsweise Dichlorbenzole, Alkylbenzole, Dialkylbenzole, Alkylnaphthaline, Alkane oder Alkene verwendet werden (siehe beispielsweise JP Hei-7-331097 und JP Hei-7-331098).
Die nach diesem Verfahren erhaltene Verbindung der allgemeinen Formel II kann in Form der dabei erhaltenen Suspension direkt, das heißt ohne Zwischenisolierung, der erfindungsgemäßen Ringschlußreaktion unterworfen werden.

Die vorliegende Erfindung betrifft auch ein Verfahren zur Herstellung besagter Dioxazin-Verbindungen durch Kondensation eines 3-Amino-carbazols der allgemeinen Formel (III), 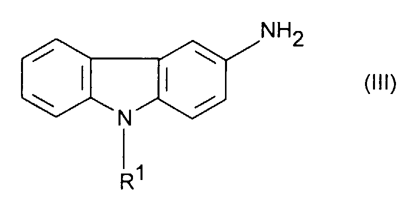 worin R¹ wie oben angegeben definiert ist, mit Chloranil in einem inerten organischen Lösungsmittel zu einem Kondensationsprodukt der allgemeinen Formel (II) 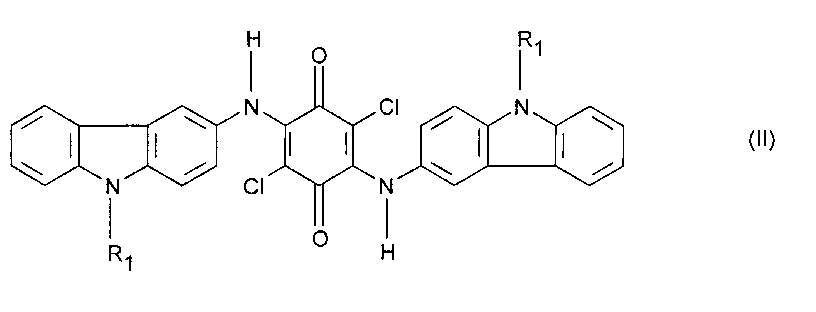 und anschließendem Ringschluß in Gegenwart eines Ringschlußmittels, dadurch gekennzeichnet, daß als Ringschlußmittel Chlor eingesetzt wird. Vorzugsweise wird das Kondensationsprodukt der allgemeinen Formel II nicht zwischenisoliert.

Als inerte organische Lösungsmittel können beispielsweise Alkylbenzole, Dialkylbenzole, Alkylnaphthaline, Alkane und Alkene eingesetzt werden. Bevorzugt ist o-Dichlorbenzol.

Die nachfolgenden Beispiele dienen der Erläuterung des erfindungsgemäßen Verfahrens.

### Beispiel 1:

In einen 2 l - Vierhalskolben mit KPG-Glasflügelrührer, Thermometer, Destillierbrücke und Ölbad gibt man 1665 g einer durch Kondensation einer technischen Lösung von 3-Amino-N-ethylcarbazol mit Chloranil erhaltenen Suspension des 2,5-Dichlor-3,6-bis(9-ethyl-3-carbazolylamino)-1,4-benzochinon in o-Dichlorbenzol. Diese Menge Suspension entspricht einem Einsatz von 126,2 g 3-Amino-N-ethyl-carbazol und 78,1 g Chloranil. Man erwärmt die Anschlämmung in 1 h unter Abdestillieren des bei der Kondensation entstandenen Reaktionswassers auf 165 °C. Bei 165 °C beginnt man mit dem Einleiten eines konstanten Chlorstroms von 19 g / h. Unter Abdestillieren steigt die Temperatur des Reaktionsgemischs in ca. 10 Minuten auf >170 °C. Während Chlor eingeleitet wird, destillieren 115 g Destillat in die Vorlage. Nach 180 Minuten wird das Einleiten von Chlor beendet. Durch Zurückwiegen der Chlorbombe und der Einleitungsapparatur wird kontrolliert und bestätigt, daß 57 g Chlor eingeleitet wurden. Das Reaktionsgemisch wird heiß filtriert. Die Paste wird mit 1500 g 160 °C heißem o-Dichlorbenzol gewaschen und bei 100 °C im Vakuum getrocknet. Die getrocknete Masse wird in 2500 g Wasser bei ca. 60 °C 1 h verrührt, filtriert und mit Wasser gewaschen. Nach dem Trocknen erhält man 142,7 g der Dioxazin-Verbindung (80,7 % der Theorie, bezogen auf 3-Amino-N-ethyl-carbazol).
Elementaranalyse: C: 70,3 %, H: 4,0 %, N: 9,2 %, O: 5,5 %, Cl: 11,5 %
Das Produkt unterscheidet sich im IR-Absorptionsspektrum nicht von dem Produkt aus dem Vergleichsbeispiel.

### Beispiel 2:

In einen 2 l-Vierhalskolben mit KPG-Glasflügelrührer, Thermometer, Destillierbrücke und Ölbad gibt man 1665 g einer durch Kondensation einer techn. Lösung von 3-Amino-N-ethylcarbazol mit Chloranil erhaltenen Suspension des 2,5-Dichlor-3,6-bis(9-ethyl-3-carbazolylamino)-1,4-benzochinon in o-Dichlorbenzol. Diese Menge Suspension entspricht einem Einsatz von 126,2 g 3-Amino-N-ethyl-carbazol und 78,1 g Chloranil. Man erwärmt die Anschlämmung in 1 h unter Abdestillieren des bei der Kondensation entstandenen Reaktionswassers auf 165 °C. Bei 165 °C beginnt man mit dem Einleiten eines Chlorstroms, der nach der folgenden Tabelle alle 20 Minuten verändert wird:

| Zeitintervall [min] | Chlorstrom [g / h] |
|---|---|
| 0 - 20 | 35,9 |
| 20 - 40 | 31,7 |
| 40 - 60 | 27,5 |
| 60 - 80 | 23,3 |
| 80 -100 | 19,0 |
| 100 -120 | 14,8 |
| 120 -140 | 10,6 |
| 140 -160 | 6,4 |
| 160 -180 | 2,2 |

Unter Abdestillieren steigt die Temperatur des Reaktionsgemischs in ca. 10 Minuten auf >170 °C. Während Chlor eingeleitet wird, destillieren 104 g Destillat in die Vorlage. Nach 180 Minuten wird das Einleiten von Chlor beendet. Durch Zurückwiegen der Chlorbombe und der Einleitungsapparatur wird kontrolliert und bestätigt, daß 57 g Chlor eingeleitet wurden. Das Reaktionsgemisch wird heiß filtriert. Die Paste wird mit 1500 g 160 °C heißem o-Dichlorbenzol gewaschen und bei 100 °C im Vakuum getrocknet. Die getrocknete Masse wird in 2500 g Wasser bei ca.60 °C 1 h verrührt, filtriert und mit Wasser gewaschen. Nach dem Trocknen erhält man 142,3 g der Dioxazin-Verbindung (80,5 % der Theorie, bezogen auf 3-Amino-N-ethyl-carbazol).
Elementaranalyse: C: 69,3 %, H: 3,9 %, N: 9,0 %, O: 5,3 %, Cl: 12,4 %
Das Produkt unterscheidet sich im IR-Absorptionsspektrum nicht von dem Produkt aus dem Vergleichsbeispiel.

### Beispiel 3:

Es wird wie in Beispiel 2 gearbeitet mit dem einzigen Unterschied, daß das Einleiten des Chlorstroms nach der folgenden Tabelle erfolgt:

| Zeitintervall [min] | Chlorstrom [g / h] |
|---|---|
| 0 - 20 | 25,8 |
| 20 - 40 | 22,8 |
| 40 - 60 | 19,8 |
| 60 - 80 | 16,7 |
| 80 -100 | 13,7 |
| 100 -120 | 10,6 |
| 120 -140 | 7,6 |
| 140 -160 | 4,6 |
| 160 -180 | 1,5 |

Während Chlor eingeleitet wird, destillieren 124 g Destillat in die Vorlage. Durch Zurückwiegen der Chlorbombe und der Einleitungsapparatur wird kontrolliert und bestätigt, daß 41 g Chlor eingeleitet wurden.
Man erhält 131,6 g der Dioxazin-Verbindung (74,4 % der Theorie, bezogen auf 3-Amino-N-ethyl-carbazol).
Elementaranalyse: C: 70,4 %, H: 3,9 %, N: 9,0 %, O: 5,0 %, Cl: 11,0 %

### Beispiel 4:

Es wird wie in Beispiel 2 gearbeitet mit dem einzigen Unterschied, daß das Einleiten des Chlorstroms nach der folgenden Tabelle erfolgt:

| Zeitintervall [min] | Chlorstrom [g / h] |
|---|---|
| 0 - 20 | 49,8 |
| 20 - 40 | 44,0 |
| 40 - 60 | 38,0 |
| 60 - 80 | 32,2 |
| 80 -100 | 26,3 |
| 100 -120 | 20,5 |
| 120 -140 | 14,6 |
| 140 -160 | 8,8 |
| 160 -180 | 3,0 |

Während Chlor eingeleitet wird, destillieren 117 g Destillat in die Vorlage. Durch Zurückwiegen der Chlorbombe und der Einleitungsapparatur wird kontrolliert und bestätigt, daß 79 g Chlor eingeleitet wurden.
Man erhält 139,0 g der Dioxazin-Verbindung (78,6 % der Theorie, bezogen auf 3-Amino-N-ethyl-carbazol).
Elementaranalyse: C: 69,7 %, H: 3,7 %, N: 8,8 %, O: 4,7 %, Cl: 12,3 %
Das Produkt unterscheidet sich im IR-Absorptionsspektrum nicht von dem Produkt aus dem Vergleichsbeispiel.

### Vergleichsbeispiel:

In einen 2 l - Vierhalskolben mit KPG-Glasflügelrührer, Thermometer, Destillierbrücke und Ölbad gibt man 1665 g einer durch Kondensation einer techn. Lösung von 3-Amino-N-ethylcarbazol mit Chloranil erhaltenen Suspension des 2,5-Dichlor-3,6-bis(9-ethyl-3-carbazolylamino)-1,4-benzochinon in o-Dichlorbenzol. Diese Menge Suspension entspricht einem Einsatz von 126,2 g 3-Amino-N-ethyl-carbazol und 78,1 g Chloranil. Man erwärmt die Anschlämmung in 1 h unter Abdestillieren des bei der Kondensation entstandenen Reaktionswassers auf 165 °C. Anschließend gibt man 70 g Benzolsulfonsäurechlorid zu und erhitzt das Gemisch in 1 h auf Siedetemperatur. Unter Abdestillieren wird 3 h nachgerührt. Es destillieren etwa 400 ml Destillat ab. Das Reaktionsgemisch wird heiß filtriert. Die Paste wird mit 1500 g 160 °C heißem o-Dichlorbenzol gewaschen und bei 100 °C im Vakuum getrocknet. Die getrocknete Masse wird in 2500 g Wasser bei ca.60 °C 1 h verrührt, filtriert und mit Wasser gewaschen. Nach dem Trocknen erhält man 150,2 g der Dioxazin-Verbindung (84,9 % der Theorie, bezogen auf 3-Amino-N-ethyl-carbazol).
Elementaranalyse: C: 69,5 %, H: 3,9 %, N: 8,8 %, O: 5,5 %, Cl: 12,1 %

Vergleich der nach dem Eindampfen der Filtrate anfallenden Abfallmengen:

| | Abfallmenge | |
|---|---|---|
| | g | kg pro t Produkt |
| Beispiel 2 | 80,7 | 567 |
| Vergleichsbeispiel | 96,8 | 664 |

### Anwendungsbeispiel 1:

In einen Kunststoffbehälter, der mit 1400 Teilen zylindrischer Mahlkörper (Cylpebs®, Durchmesser 12 mm, Hersteller: Groh GmbH, Hof) zu 90 Vol-% gefüllt ist, werden nacheinander 22,5 Teile der Dioxazin-Verbindung hergestellt gemäß einem der vorstehenden Beispiele, und 7,5 Teile wasserfreies Natriumsulfat eingebracht. Die Mischung wird 4 Stunden lang unter Schütteln auf einer Schwingmühle (Typ Vibratom; Hersteller: Siebtechnik Mühlheim) fein vermahlen. Danach wird das Mahlgut von den Mahlkörpern abgesiebt. Man erhält 25,9 Teile Mahlgut. In einem Rührgefäß werden 37,5 Teile Isobutanol (85 %ig) vorgelegt und nacheinander 2,5 Teile Ameisensäure 98 %ig und 0,38 Teile des Natriumsalzes eines Alkylphenolpolyglykolethersulfats zugegeben. Danach werden 25 Teile des obigen Mahlguts eingetragen und 20 Stunden bei 20-25°C gerührt. Während dieser Zeit werden noch 50 Teile Isobutanol 85 %ig zugesetzt. Anschließend werden 150 Teile Wasser zugegeben, 5 Stunden zum Sieden erhitzt und das Isobutanol durch Erhitzen auf 100 °C am Übergang azeotrop abdestilliert. Nach dem Abkühlen auf 60°C wird das Pigment abgesaugt, mit Wasser neutral und salzfrei gewaschen und bei 80°C getrocknet.
Man erhält 18,6 Teile Pigment, das im Alkydmelaminharz-Lack transparente und farbstarke violette Lackierungen mit roter Nuance liefert. Die Überlackierechtheit ist einwandfrei. Im Nitrocellulosetiefdruck werden transparente und farbstarke Drucke mit hohem Glanz erhalten.

### Anwendungsbeispiel 2:

In einen Kunststoffbehälter, der mit 1400 Teilen zylindrischer Mahlkörper (Cylpebs®, Durchmesser 12 mm, Hersteller: Groh GmbH, Hof) zu 90 Vol-% gefüllt ist, werden 30 Teile der Dioxazin-Verbindung, hergestellt gemäß einem der vorstehenden Beispiele, eingebracht. Das Rohpigment wird 4 Stunden lang unter Schütteln auf einer Schwingmühle (Typ Vibratom; Hersteller: Siebtechnik Mühlheim) fein vermahlen. Danach wird das Mahlgut von den Mahlkörpern abgesiebt. Man erhält 25,9 Teile Mahlgut.
In einem Rührgefäß werden 37,5 Teile Isobutanol (85 %ig) vorgelegt und 1,25 Teile Ameisensäure 98 %ig zugegeben. Danach werden 25 Teile des obigen Mahlguts eingetragen und 20 Stunden bei 20-25°C gerührt. Während dieser Zeit werden noch 50 Teile Isobutanol 85 %ig zugesetzt. Anschließend werden 150 Teile Wasser zugegeben und das Isobutanol durch Erhitzen auf 100°C am Übergang azeotrop abdestilliert. Nach dem Abkühlen auf 60°C wird das Pigment abgesaugt, mit Wasser neutral gewaschen und bei 80°C getrocknet.
Man erhält 24,2 Teile Pigment, das in PVC transparente und farbstarke violette Ausfärbungen liefert. Die Ausblutechtheit ist sehr gut.

## Patentansprüche

1. Verfahren zur Herstellung von Dioxazin-Verbindungen durch Ringschluß einer Verbindung der allgemeinen Formel (II) 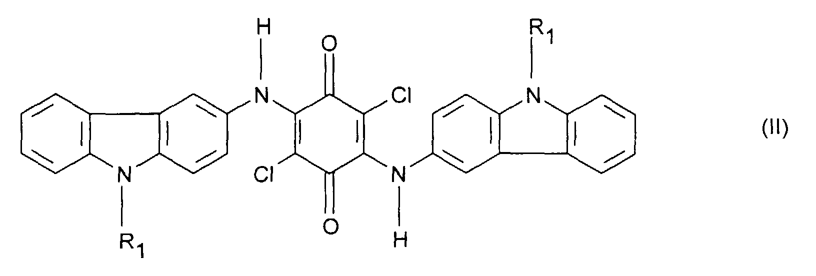 worin R¹ Wasserstoff oder (C₁-C₈)-Alkyl bedeutet, in Gegenwart eines Ringschlußmittels, dadurch gekennzeichnet, daß als Ringschlußmittel Chlor eingesetzt wird.

2. Verfahren gemäß Anspruch 1, dadurch gekennzeichnet, daß R¹ für Ethyl steht.

3. Verfahren zur Herstellung von Dioxazin-Verbindungen durch Kondensation eines 3-Amino-carbazols der allgemeinen Formel (III) 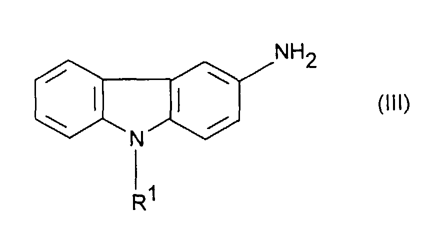 worin R¹ Wasserstoff oder (C₁-C₈)-Alkyl bedeutet, mit Chloranil in einem inerten organischen Lösungsmittel zu einem Kondensationsprodukt der allgemeinen Formel (II) 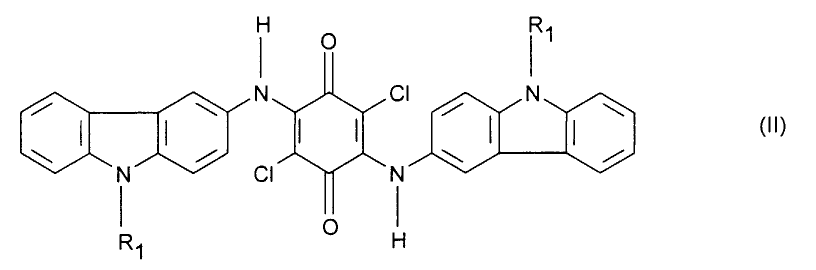 und anschließendem Ringschluß in Gegenwart eines Ringschlußmittels, dadurch gekennzeichnet, daß als Ringschlußmittel Chlor eingesetzt wird.

4. Verfahren gemäß Anspruch 3, dadurch gekennzeichnet, daß die Verbindung der allgemeinen Formel II nicht zwischenisoliert wird.

5. Verfahren gemäß einem oder mehreren der Ansprüche 1 bis 4, dadurch gekennzeichnet, daß das Chlor in Mengen von 1,5 bis 5 Mol pro Mol Verbindung der allgemeinen Formel II eingesetzt wird.

6. Verfahren gemäß einem oder mehreren der Ansprüche 1 bis 5, dadurch gekennzeichnet, daß das Chlor in Mengen von 2,2 bis 3,5 Mol pro Mol Verbindung der allgemeinen Formel II eingesetzt wird.

7. Verfahren gemäß einem oder mehreren der Ansprüche 1 bis 6, dadurch gekennzeichnet, daß der Ringschluß bei einer Temperatur zwischen 150 und 200°C erfolgt.

8. Verfahren gemäß einem oder mehreren der Ansprüche 1 bis 7, dadurch gekennzeichnet, daß der Ringschluß in Gegenwart eines inerten organischen Lösungsmittels durchgeführt wird.

9. Verfahren gemäß Anspruch 8, dadurch gekennzeichnet, daß das inerte organische Lösungsmittel ein Alkylbenzol, Dialkylbenzol, Alkylnaphthalin, Alkan, Alken, o-Dichlorbenzol, m-Dichlorbenzol oder p-Dichlorbenzol ist.
